Europäisches Patentamt

European Patent Office

Office européen des brevets

(11) Veröffentlichungsnummer: **0 379 658**
**A2**

(12) **EUROPÄISCHE PATENTANMELDUNG**

(21) Anmeldenummer: **89120739.1**

(22) Anmeldetag: **09.11.89**

(51) Int. Cl.5: **C11D 1/825, C11D 1/83,**
**C11D 1/94, C11D 1/66**

(30) Priorität: **27.01.89 DE 3902374**

(43) Veröffentlichungstag der Anmeldung:
**01.08.90 Patentblatt 90/31**

(84) Benannte Vertragsstaaten:
**AT BE CH DE ES FR GB GR IT LI LU NL SE**

(71) Anmelder: **DEUTSCHE SOLVAY-WERKE GMBH**
**Langhansstrasse 6 Postfach 11 02 70**
**D-5650 Solingen 11(DE)**

(72) Erfinder: **Jakobson, Gerald, Dr.**
**Willinger Weg 21**
**D-4134 Rheinberg 2(DE)**
Erfinder: **Siemanowski, Werner, Dr.**
**Am Annaberg 18**
**D-4134 Rheinberg 1(DE)**
Erfinder: **Uhlig, Karl-Heinz**
**Waldesheimer Weg 66**
**D-4150 Krefeld 1(DE)**

(74) Vertreter: **Seiler, Siegfried**
**Langhansstrasse 6**
**D-5650 Solingen 11(DE)**

(54) **Wasch-, Reinigungs- und/oder Körperreinigungsmittel.**

(57) Die vorliegende Erfindung betrifft ein Wasch-, Reinigungs- und/oder Körperreinigungsmittel, enthaltend mindestens ein ionogenes und/oder amphoteres Tensid und mindestens einen Monofettsäureester mit $C_8$ bis $C_{18}$ des Diglycerins und/oder einen Difettsäureester mit $C_8$ bis $C_{18}$ des Tetraglycerins als Mischungsbestandteil, wobei in dem das Tensidgemisch 2 bis 30 Gew.-%, vorzugsweise 10 bis 20 Gew.-%, mindestens eines Monofettsäureesters des Diglycerins und/oder Difettsäureesters des Tetraglycerins, bezogen auf den Gesamttensidgehalt (100 Gew.-%) enthalten sind. Das Mittel wird mit bestimmten Zusammensetzungen, insbesondere mit Lösemittel, Zusatzstoffen und dgl. eingesetzt.

Die Erfindung betrifft weiterhin die Herstellung des Wasch-, Reinigungs- und/oder Körperreinigungsmittels sowie dessen Verwendung als Dusch-, Schaumbademittel, flüssiges Handreinigungsmittel oder Haarshampoo.

EP 0 379 658 A2

EP 0 379 658 A2

Die vorliegende Erfindung betrifft ein Wasch-, Reinigungs- und/oder Körperreinigungsmittel, enthaltend mindestens ein nichtionogenes, aus einem Fettsäureester bestehendes Tensid und mindestens ein ionogenes und/oder amphoteres Tensid. Die Erfindung betrifft weiterhin das Verfahren zur Herstellung des Wasch-, Reinigungs- und/ oder Körperreinigungsmittels sowie die Verwendung desselben.

Aus der DE-PS 30 33 929 ist bereits ein Körperreinigungsmittel auf der Basis einer wäßrigen Lösung eines Gemisches von bestimmten Betainen und einer oder mehreren anionischen Verbindungen aus der Gruppe Natrium- oder Ammoniumalkylethersulfat, Alkanolaminethersulfat, Alkanolaminalkylsulfat, wobei die Alkylgruppe 8 bis 14 Kohlenstoffatome aufweist und bestimmte Gewichtsverhältnisse einzuhalten sind, bekannt, wobei zusätzlich zwei bis 35 Gew.-%, (bezogen auf das Betain) Glycerinfettsäureester mit einem Mindestanteil von 70 Gew.-% Monoester, deren Fettsäurekomponente 8 bis 18 Kohlenstoffatome aufweist, eingesetzt werden.

Dieses Körperreinigungsmittel weist jedoch die Nachteile auf, daß der in der wäßrigen Lösung enthaltene Fettsäureester, insbesondere Glycerinmonofettsäureester, in der wäßrigen Lösung des Gesamttensidgemisches relativ schwer löslich ist. Dieser Sachverhalt macht sich vor allem dann bemerkbar, wenn der Gesamttensidgehalt in der wäßrigen Lösung vermindert wird, so daß Trübungen und Wirkungsverminderungen auftreten. Weiterhin ist es erforderlich, den Glycerinfettsäureester bei Temperaturen über ca. 60 °C aufzuschmelzen und nachfolgend zu verarbeiten oder den Glycerinfettsäureester mit Lösemittel, Lösungsvermittler oder Dispergiermittel in Lösung oder Dispersion zu bringen. Bei den Glycerinmonofettsäureestern sind Emulgier- und Solubisierungseigenschaften, wie sie z. B. zur Auflösung von Parfümölen und/oder anderen Wirkstoffen zweckmäßig sind, nur schwach ausgebildet.

Ziel und Aufgabe der vorliegenden Erfindung war es daher die vorgenannten Nachteile zu vermeiden.

Insbesondere sollte die Wirksamkeit und/oder Verarbeitbarkeit des Mittels verbessert werden. Das Mittel sollte als nichtionogenes Tensid eine chemische Verbindung aufweisen, die den dermatologischen und toxikologischen Anforderungen entspricht, insbesondere hautverträglich sein und in den Anwendungskonzentrationen keine toxischen Eigenschaften aufweisen. Das nichtionogene Tensid sollte biologisch leicht abbaubar sein.

Erfindungsgemäß wurde festgestellt, daß diesen Zielen und Aufgaben ein Wasch-, Reinigungs- und/oder Körperreinigungsmittel, enthaltend mindestens ein nichtionogenes, aus einem Fettsäureester bestehendes Tensid und mindestens ein ionogenes und/oder amphoteres Tensid gerecht wird. Das erfindungsgemäße Tensidgemisch enthält mindestens einen Monofettsäureester mit $C_8$ bis $C_{18}$, vorzugsweise $C_9$ bis $C_{17}$, des Diglycerins und/oder einen Difettsäureester mit $C_8$ bis $C_{18}$ des Tetraglycerins als Mischungsbestandteil.

Das Tensidgemisch enthält gemäß der Erfindung 2 bis 30 Gew.-%, vorzugsweise 10 bis 20 Gew.-%, mindestens eines Monofettsäureesters des Diglycerins und/oder Difettsäureesters des Tetraglycerins, bezogen auf den Gesamttensidgehalt (100 Gew.-S). Das Wasch-, Reinigungs- und/oder Körperreinigungsmittel kann in fester, flüssiger (einschließlich gelartiger oder hochviskoser Form) oder pastöser Form vorliegen. Die flüssige Form wird bevorzugt eingesetzt.

Es war nicht zu erwarten, daß der Monofettsäureester mit $C_8$ und $C_{18}$ des Diglycerins und/oder der Difettsäureester mit $C_8$ und $G_{18}$ des Tetraglycerins in dem Tensidgemisch die gewünschten Ergebnisse erbringt und u. a. diese Verbindung auch den gestellten Anforderungen, z. B. dermatologischen sowie toxikologischen Anforderungen hinsichtlich der Wirksamkeit, entspricht, da Diglycerin und Tetraglycerin nicht als Homologe von Glycerin angesehen werden können und chemisch keine dreiwertigen Alkohole wie Glycerin darstellen, sondern Ether-Polyalkohole sind.

Das Wasch-, Reinigungs- und/oder Körperreinigungsmittel enthält bevorzugt zusätzlich Elektrolyte, vorzugsweise anorganische Chloride, in Gewichtsmengen von 0,1 bis 8 Gew.-%, vorzugsweise 0,7 bis 3 Gew.-%, bezogen auf das Gesamtgewicht des Wasch-, Reinigungs- und/oder Körperreinigungsmittels.

Der Gesamttensidgehalt des Mittels unter Verwendung des erfindungsgemäßen Tensidgemisches beträgt 2,5 bis 60 Gew.-%, vorzugsweise 7 bis 35 Gew.-%, und der Restbestandteil besteht aus Lösungs- und/oder Verdünnungsmitteln, vorzugsweise Wasser, niedrigen Alkoholen mit $C_1$ bis $C_8$, vorzugsweise $C_2$ bis $C_4$, Diolen mit $C_2$ bis $C_8$, vorzugsweise $C_3$ bis $C_6$, und/oder Glycerin oder Glycerinderivaten und mindestens einem Elektrolyten sowie ggf. Zusatzmitteln, vorzugsweise Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln oder enthält diese.

Nach einer bevorzugten Ausführungsform beträgt der Gesamttensidgehalt des Mittels 2,5 bis 60 Gew.-%, vorzugsweise 7 bis 35 Gew.-%, wobei das Tensidgemisch aus 2 bis 30 Gew.-%, vorzugsweise 10 bis 20 Gew.-%, mindestens eines Monofettsäureesters des Diglycerins und/oder Difettsäureesters des Tetraglycerins mit $C_{12}$ bis $C_{16}$ (bezogen auf den Gesamttensidgehalt 100 Gew.-%) besteht. Das Mittel enthält 7 bis 0,1 Gew.-%, vorzugsweise 3 bis 0,3 Gew.-%, mindestens eines Elektrolyten, vorzugsweise bestehend aus

2

oder enthaltend Natriumchlorid, 90,5 bis 39,9 Gew.-%, vorzugsweise 90 bis 64,7 Gew.-%, eines Lösungs- und/oder Verdünnungsmittels, vorzugsweise Wasser, niedrige Alkohole mit $C_1$ bis $C_8$, vorzugsweise $C_2$ bis $C_4$, und/oder Diole mit $C_3$ bis $C_8$, vorzugsweise $C_3$ bis $C_6$. Es enthält zusätzlich Zusatzmittel in Gewichtsmengen von 0,005 bis 12 Gew.-Teile (bezogen auf 100 Gew.-Teile des, aus dem Tensidgemisch, Elektrolyten und Lösungs- und/oder Verdünnungsmittel bestehenden Wasch-, Reinigungs- und/oder Körperreinigungsmittels), vorzugsweise 0,1 bis 5 Gew.-Teile, vorzugsweise bestehend aus Konservie rungs-, Parfümierungs-, Färbemitteln, pharmazeutischen Wirkstoffen, Stellmittel zur pH-Regulierung, Komplexierungsmittel zur Maskierung von Metallionen, Hautpflegemitteln und/oder Verdickungsmitteln.

Der Monofettsäureester mit $C_8$ bis $C_{18}$, vorzugsweise $C_{12}$ bis $C_{16}$, des Diglycerins und/oder der Difettsäureester mit $C_8$ bis $C_{18}$, vorzugsweise $C_{12}$ bis $C_{16}$, des Tetraglycerins erbringt in dem Tensidgemisch für das Körperreinigungsmittel neben milderer Reinigungswirkung bestimmte zusätzliche Eigenschaften, wie z. B. einen Rückfettungseffekt und ein angenehmes Hautgefühl während und nach dem Reinigungsvorgang, ein verbessertes Fließverhalten und weist darüber hinaus eine dermatologische und toxikologische Unbedenklichkeit auf. Unter Verwendung des Monofettsäureesters mit $C_8$ bis $C_{18}$, vorzugsweise $C_{12}$ bis $C_{16}$, des Diglycerins und/oder Difettsäureesters mit $C_8$ bis $C_{18}$, vorzugsweise $C_{12}$ bis $C_{16}$, des Tetraglycerins gelingt es beispielsweise eine wichtige Substanzklasse für Reinigungsund/oder Körperwaschmittel, nämlich die Fettsäurealkanolamide, speziell Kokosfettsäureethanolamid zu ersetzen, da diese aufgrund u. a. ihrer toxikologischen Bedenklichkeit (Verdacht der Bildung von Nitrosaminen) in den Reinigungs- und Körperwaschmitteln z. T. nicht mehr erwünscht sind.

Es gelingt auch, in den Reinigungs- und Körperwaschmitteln Glycerin-Monolaurat oder andere Glycerin- Monofettsäureester zu ersetzen. Nachteilig ist bei den Glycerin-Monofettsäureestern, insbesondere bei Glycerin-Monolaurat, der verhältnismäßig hohe Schmelzpunkt (z. B. von 60 °C) und die relativ schlechte Löslichkeit dieser Verbindungen, so daß eine Anwendung von Glycerin-Monofettsäureestern nur in vorher dispergierter Form sinnvoll oder zweckmäßig ist.

Nach einer bevorzugten Ausführungsform enthält das Tensidgemisch 2 bis 30 Gew.-% Diglycerin- Monolaurat (bezogen auf den Gesamttensidgehalt 100 Gew.-%).

Nach einer bevorzugten Ausführungsform enthält das Wasch- und Reinigungs- und/oder Körperreinigungsmittel mindestens ein ionogenes Tensid, bestehend aus mindestens einem Alkylaryl- oder Alkylethersulfat, Alkylhydroxyethersulfonat, Alkylsulfat, Alkylarylsulfonat, vorzugsweise Alkylbenzolsulfonat, Acylaminopolyglykolether-Sulfat, Olefinsulfonat, Paraffinsulfonat, Sulfo-Bernsteinsäureester und/oder Fettalkoholether-Carboxylat und/oder als amphoteres Tensid Alkylamido-Betain und/oder ein amphoteres Glycerinderivat.

Nach einer weiteren bevorzugten Ausführungsform enthält das Wasch-, Reinigungs- und/oder Körperreinigungsmittel ein Alkylethersulfat, Alkylethersulfonat, Alkylhydroxyethersulfonat, Aralkylethersulfonat und/oder Alkylsulfat. Als Kation enthalten die Tensinde $Na^+$, $K^+$, $Mg^{++}$, $NH_4^+$, Alkylammonium, Alkanolam- monium, vorzugsweise Monoethanolammonium, Triethanolammonium $Na^+$ und/oder $NH_4^+$.

Nach einer bevorzugten Ausführungsform hat das Wasch-, Reinigungs- und/oder Körperreinigungsmittel einen Gesamttensidgehalt von 2,5 bis 60 Gew.-%, vorzugsweise 7 bis 35 Gew.-%, (bezogen auf 100 Gew.- % des Mittels), wobei das Tensidgemisch 2 bis 30 Gew.-%, vorzugsweise 10 bis 20 Gew.-%, mindestens eines Monofettsäureesters des Diglycerins und/oder Difettsäureesters des Tetraglycerins, bezogen auf den Gesamttensidgehalt (100 Gew.-%) und 98 bis bis 70 Gew.%, vorzugsweise 90 bis 80, mindestens eines ionogenen Tensides, bestehend aus mindestens einem Alkylaryl- oder Alkylethersulfat, Alkylsulfat, Alkylethersulfonat, Alkylhydroxyethersulfonat, Polyhydroxyalkylethersulfonat, Alkylarylsulfonat, vorzugsweise Alkylbenzolsulfonat, Acylaminopolyglykolether-Sulfat, Olefinsulfonat, Paraffinsulfonat, Sulfo-Bernsteinsäureester und/oder Fettalkoholether-Carboxylat und/oder als amphoteres Tensid Alkylamido-Betain und/oder amphoteres Glycerinderivat, vorzugsweise jedoch ein ionogenes Tensid oder Tensidgemisch enthaltend oder bestehend aus Alkylethersulfat, Alkylhydroxyethersulfonat, Alkylsulfonat und/oder Alkylsulfat, das als Kation $Na^+$, $K^+$, $Mg^{++}$, $NH_4^+$, Monoethanolammonium und/oder Triethanolammonium enthält, und der Restbestandteil aus Lösungs- und/oder Verdünnungsmitteln, vorzugsweise Wasser, niedrige Alkohole mit $C_1$ bis $C_8$, vorzugsweise $C_2$ bis $C_4$, und/oder Glycerin oder Glycerinderivate, Elektrolyte sowie ggf. Zusatzmittel, vorzugsweise Konservierungs-, Parfümierungs-, Färbemittel, pharmazeutische Wirkstoffe, Stellmittel zur pH- Regulierung, Komplexierungsmittel zur Maskierung von Metallionen, Hautpflegemittel und/oder Verdickungsmittel enthält.

Nach einer weiteren bevorzugten Ausführungsform enthält dieses Mittel 7 bis 0,1 Gew.-%, vorzugsweise 3 bis 0,3 Gew.-%, mindestens eines Elektrolyten, vorzugsweise bestehend aus oder enthaltend Natriumchlorid und/oder Ammoniumchlorid, 90,5 bis 39,9 Gew.-%, vorzugsweise 90 bis 64,7 Gew.-%, eines Lösungs- und/oder Verdünnungsmittels, vorzugsweise Wasser, niedrige Alkohole mit $C_1$ bis $C_8$, vorzugswei- se $C_2$ bis $C_4$, und/oder Diole mit $C_3$ bis $C_8$, vorzugsweise $C_3$ bis $C_6$, und Zusatzmittel in Gewichtsmengen von 0,005 bis 12 Gew.-Teile (bezogen auf 100 Gew.-Teile des, aus dem Tensidgemisch, Elektrolyten und Lösungs- und/oder Verdünnungsmittels bestehenden Wasch-, Reinigungs- und/oder Körperreinigungsmittel), vorzugsweise 0,1 bis 5 Gew.-Teile, vorzugsweise bestehend aus Konservierungs-, Parfümierungs-, Färbemitteln, pharmazeutischen Wirkstoffen, Stellmittel zur pH-Regulierung, Komplexierungsmittel zur Maskierung von Metallionen, Hautpflegemitteln und/oder Verdickungsmitteln.

Als Alkylethersulfate, Alkylarylsulfonate, Alkylsulfate und, Alkylsulfonate, deren Ester und Olefinsulfonate werden bevorzugt solche mit einer C-Zahl von 8 -22, vorzugsweise 10 - 18, eingesetzt. Bei diesen mit einem hydrophoben Rest in Form eines gesättigten, ungesättigten oder verzweigten Kohlenwasserstoffrest versehenen Tensides kann der hydrophobe Rest auch über einen Phenylrest und/oder über andere Heteroatome, z. B. Sauerstoff mit der Sulfat- und/oder Sulfonatgruppe verbunden sein, z. B. als Ätherethylsulfate. Bevorzugt werden Alkali-, insbesondere Natriumlaurylethersulfat, Acylaminopolyglykolethersulfat, vorzugsweise in Form der Alkanolaminverbindung, insbesondere Triethanolaminsalz, Sulfobernsteinsäureester oder Sulfodicarbonsäureester, Alkali-Benzolsulfonat und Ammoniumalkylbenzolsulfonat, vorzugsweise Natrium- oder Ammoniumalkylbenzosulfonat oder Triethanolammonium-Alkylbenzolsulfonat und dgl. eingesetzt.

Als Alkyläthersulfate werden bevorzugt Verbindungen der allgemeinen Formel $RO(C_2H_4O)_nSO_3M$, in der R eine Alkylkette mit 10 - 18, vorzugsweise 12 - 14, Kohlen stoffatomen, n Zahlen von 1 bis 10 und M ein Kation darstellen, zu. Diese Alkyläthersulfate werden durch Äthoxylierung einwertiger Akohole mit 10 - 18 Kohlenstoffatomen bis zum gewünschten Äthoxylierungsgrad und nachfolgender Sulfatierung und/oder Neutralisation gewonnen. Die Neutralisation der sauren Sulfatierungsprodukte oder der Sulfonate kann durch Alkalien, Ammoniak, Amine oder Alkanolamine und/oder durch Magnesiumhydroxid erfolgen. Als Alkylhydroxyethersulfonat oder Polyhydroxyalkylethersulfat werden bevorzugt solche mit $C_{10}$ - $C_{18}$, vorzugsweise $C_{12}$ - $C_{14}$, in der Fettsäurekette eingesetzt.

Als Elektrolyte können neben Natrium- und/oder Ammoniumchlorid auch an sich in Körperreinigungsmitteln eingesetzte Elektrolyte zum Einsatz gelangen.

Weiterhin können die Reinigungs, Wasch- und/oder Körperreinigungsmittel andere Verbindungen wie Kolloide, wie Derivate der Cellulose oder Stärke, desinfizierende Wirkstoffe, fungizide oder antibakterielle Wirkstoffe, Korrosionsschutzmittel usw. enthalten.

Die Erfindung betrifft weiterhin die Verwendung des Wasch-, Reinigungs- und/oder Körperreinigungsmittels als Duschgel oder Duschmittel, Schaumbademittel, flüssiges Handreinigungsmittel oder Haarshampoo.

Die Erfindung betrifft auch ein Verfahren zur Herstellung von Wasch-, Reinigungs- und/oder Körperreinigungsmitteln enthaltend mindestens ein ionogenes und ein nichtionogenes Tensid, von denen das nichtionogene Tensid unter Verwendung von Fettsäurealkylestern, Mono- oder Polyhydroxyfettsäurealkylestern mit $C_8$ - $C_{18}$ in der Fettsäurekomponente und $C_1$ - $C_4$ in der Esterkomponente, die im alkalischen Medium mit einem oder mehreren Isopropylidenderivaten eines Polyglycerins umgesetzt werden, hergestellt wird. Diese Umsetzung wird mit einem Di- und/oder Tetraglycerin bei Temperaturen von 140 - 220 °C, vorzugsweise 170 - 200 °C, und im Vakuum bei 950 - 5 mbar, vorzugsweise bei 500 bis 10 bar, durchgeführt, der dabei entstehende $C_1$ bis $C_4$ Alkohol durch Destillation entfernt, vorzugsweise kontinuierlich entfernt und das nachfolgend gereinigte, vorzugsweise durch Filtration, Zentrifugation, Destillation und/oder fraktionierte Destillation gereinigte Reaktionsprodukt hydrolisiert wird, indem mindestens eine Isopropylidengruppe des Reaktionsproduktes bei 20 bis 100 °C, vorzugsweise 50 bis 80 °C, sowie bei Normal-, Unter- oder Überdruck durch saure Hydrolyse gespalten wird. Die entstandenen Monofettsäureester des Diglycerins und/oder Tetraglycerins werden bei Temperaturen von 1 bis 80 °C, vorzugsweise 10 bis 40 °C, und Druck oder Unterdruck von 0,1 bis 1,5 bar, vorzugsweise 0,3 bis 1,2 bar, mit mindestens einem ionogenen Tensid, vorzugsweise mindestens einem Alkylethersulfat, Alkylethersulfonat, Alkylhydroxetherysulfonat, Polyhydroxyalkylethersulfonat, Alkylsulfat, Alkylbenzolsulfonat, Acylaminopolyglykolether-Sulfat, Olefinsulfonat, Paraffinsulfonat, Sulfo-Bernsteinsäureester, Alkylamido-Betain, amphoteren Glycinderivat und/oder Fettalkoholether-Carboxylat, und/oder den anderen Bestandteilen oder einem Teil derselben vermischt und/oder nachfolgend die restlichen Bestandteile hinzugefügt.

Bevorzugt wird bei dem Verfahren zur Herstellung dieses nichtionogenen Tensides ein Überschuß von Diglyce rin zur Umsetzung eingesetzt und/oder ein entstehendes Gemisch an Di- und Monofettsäureestern des Diglycerins destillativ aufgetrennt.

Das Wasch-, Reinigungs- und/oder Körperreinigungsmittel wird auf einen pH-Wert von 2 - 8, vorzugsweise 4 -7, eingestellt.

Der in dem Tensidgemisch eingesetzte Monofettsäureester mit $C_8$ bis $C_{18}$, vorzugsweise $C_{12}$ bis $C_{16}$, des Diglycerins und/oder der Difettsäureester mit G8 bis $C_{18}$, vorzugsweise $C_{12}$ bis $C_{16}$, des Tetraglycerins wirkt in dem Wasch-, Reinigungs und/oder Körperreinigungsmittel gleichzeitig als Verdickungs- oder

4

EP 0 379 658 A2

Viskositätssteigerungsmittel. Verwendet man beispielsweise eine 18gew.-%ige Lösung von Natriumlaurylethersulfat, d. h. einen Gewichtsanteil von 18 Gew.-% Natriumlaurylethersulfat in der Lösung, ohne Mitverwendung von anderen Tensiden oder Zusätzen, jedoch mit einem Gehalt von 1,5 % Natriumchlorid, so ergibt sich eine Viskosität von 169 mPas. Werden 10 Gew.-% des Natriumlaurylethersulfates, d. h. 1,8 Gew.-Teile pro 100 Gew.-Teile der Lösung durch die gleiche Gewichtsmenge, nämlich 1,8 Gew.-Teile Diglycerinmonolaurat ersetzt, so ergibt sich eine Viskosität von 1.270 mPas bei 1,5 % Natriumchloridgehalt oder 12.000 mPas bei 2gew.-%igen Natriumchloridgehalt. Werden 15 Gew.-% des Natriumlaurylethersulfates, d. h. 2,7 Gew.-Teile pro 100 Gew.-Teile der Lösung durch die gleiche Gewichtsmenge Diglycerinmonolaurat ersetzt, so ergibt sich eine Viskosität von 6.200 mPas bei einem Natriumchloridgehalt von 1,5 Gew.-%.

Ersetzt man 20 Gew.-% des eingesetzten Natriumlauryl ethersulfates (bei einer eingesetzten Gewichtsmenge von 18 Gew.-%, d. h. ersetzte Gewichtsmenge 3,6 Gew.-% oder 3,6 Gew.-Teile, bezogen auf 100 Gew.-Teile der Lösung) durch die gleiche Gewichtsmenge, nämlich 3,6 Gew.-Teile Diglycerinmonolaurat, so ergibt sich eine Viskosität von 13.000 mPas bei einem Natriumchloridgehalt von 1,5 Gew.-%.

Werden ausgehend von einer wäßrigen Lösung von 18 Gew.-% Natriumlaurylethersulfat 10 Gew.-% des eingesetzten Natriumlaurylethersulfates, d. h. 1,8 Gew.-Teile durch die gleiche Gewichtsmenge Diglycerinmonopalmitat, so daß die angesetzte Lösung 16,2 Gew.-% Natriumlaurylethersulfat und 1,8 Gew.-% Diglycerinmonopalmitat enthält, so ergibt sich eine Viskosität von 704 mPas bei einem Natriumchloridgehalt von 1,5 Gew.-% und 7.320 mPas bei einem Natriumchloridgehalt von 9,0 Gew.-%.

Werden 15 Gew.-% des Natriumlaurylethersulfates (bezogen auf 18 Gew.-% Natriumlaurylethersulfat-Lösung, d. h. 2,7 Gew.-Teile durch die gleiche Gewichtsmenge Diglycerin-Monopalmitat ersetzt, erhält man eine 15,3gew.-%ige Natriumlaurylethersulfat-Lösung, die 2,7 Gew.-% Diglycerinmonopalmitat enthält und es ergibt sich eine Viskosität von 2.250 mPas bei 1,5 Gew.-% Natriumchloridgehalt.

Unter Mitverwendung der erfindungsgemäßen Monofettsäureester des Diglycerins und Difettsäureester des Tetraglycerins gelingt es auch, das Schaumverhalten der Wasch-, Reinigungs- und/oder Körperreinigungsmittel zu verbessern. Bei Waschversuchen in der Praxis wurde darüber hinaus bei der Verwendung als Haarshampoo während des Shampooniervorganges ein angenehmeres cremiges Verhalten festgestellt.

Darüber hinaus wird bei der Verwendung des erfindungsgemäßen Wasch-, Reinigungs- und/oder Körperreinigungsmittels ein sehr guter Rückfettungseffekt erhalten. Die eingesetzten Monofettsäureester mit $C_8$ bis $C_{18}$, vorzugsweise $C_{12}$ bis $C_{16}$, des Diglycerins und/oder Difettsäureester mit $C_8$ bis $C_{18}$, vorzugsweise $C_{12}$ bis $C_{16}$, des Tetraglycerins sind als reine definierte Verbindungen frei von unerwünschten Nebenprodukten herstellbar und zählen in mehreren Ländern zu der Verbindungsklasse der Polyglycerinfettsäureester, die nahrungsmittelrechtlich zugelassen ist. Diese Verbindungen sind zu mehr als 80 % biologisch abbaubar (Sekundärabbau gemessen über CSB-Abnahme).

Die eingesetzten Monofettsäureester des Diglycerins und/oder Difettsäureester des Tetraglycerins sind von schmalzartiger Konsistenz, können daher gut eingearbeitet werden und sind in den üblichen Tensidkonzentrationen leicht oder klar löslich.

Beispiele

| 1. Haarshampoo-Standardrezepturen | |
|---|---|
| (Gesamttensidgehalt ca. 18 Gew.-%) | |
| Natriumlaurylethersulfat 70%ig | 21,9 Gew.-% |
| Diglycerinmonolaurat | 2,7 Gew.-% |
| Natriumchlorid | 1,5 Gew.-% |
| Konservierungsmittel | 0,05 Gew.-% |
| Parfüm | 0,2 Gew.-% |
| Wasser voll entsalzt | 73,65 Gew.-% |

Viskosität: 6.200 mPas
Schaumzahlen nach Ross/Miles DIN 53902
1 g/l WAS, 40 °C, dest. $H_2O$ : 163/155
Schaumverhalten:

5

Es wurde gemessen nach Ross/Miles in einer 1 g/l Aktivsubstanzlösung in destl. $H_2O$ bei 40 °C.
Folgende Schaumwerte wurden festgestellt:
mit Zusatz von Monofettsäueester des Diglycerins wie unter 1. beschrieben

| Schaumhöhe | sofort | 163 mm |
|---|---|---|
| | nach 5 sec | 155 mm |

(Durchschnittswerte) gegenüber Natriumlaurylethersulfat 18 Gew.-% WAS (waschaktive Substanz, d. h. Gesamt-Tensidgehalt) ohne Zusatz

| Schaumhöhe | sofort | 139 mm |
|---|---|---|
| | nach 5 sec | 130 mm |

Dies bedeutet eine Zunahme von durchschnittlich 17 % an Schaumvolumen durch Einsatz von Monofettsäureestern des Diglycerins.

| 2. Haarshampoo-Standardrezepturen | |
|---|---|
| (Gesamt-Tensidgehalt ca. 18 Gew.-%) | |
| Natriumlaurylethersulfat 70%ig | 21,9 Gew.-% |
| Diglycerinmonolaurat | 2,7 Gew.-% |
| Natriumchlorid | 1,5 Gew.-% |
| Konservierungsmittel | 0,05 Gew.-% |
| Parfüm | 0,2 Gew.-% |
| Wasser voll entsalzt | 73,65 Gew.-% |

Viskosität: 11.000 mPas
Schaumzahlen nach Ross/Miles DIN 53902
1 g/l WAS, 40 °C, dest. $H_2O$ : 165/158
Viskosität gemessen nach Rotationsviskosimeter VT 181 der Firma Haake bei 20 °C.

| 3. Rezeptur für ein hautmildes Körperreinigungsmittel und hautmildes Haarshampoo | |
|---|---|
| (ca. 22 % Gesamt-Tensidgehalt) | |
| Natriumlaurylethersulfat 70%ig | 13,0 Gew.-% |
| Di-Natriumfettalkoholpolyglykolether-Sulfosuccinat 40%ig (Sulfobernsteinsäueester) | 25,5 Gew.-% |
| Diglycerinmonolaurat | 2,7 Gew.-% |
| Natriumchlorid | 4,0 Gew.-% |
| Wasser vollentsalzt | 54,8 Gew.-% |

Viskosität: 2.300 mPas
Schaumzahlen nach Ross/Miles DIN 53902
1 g/l WAS, 40 °C, dest. $H_2O$ : 165/156

| 4. Rezeptur für ein hautmildes Körperreinigungsmittel und hautmildes Haarshampoo | |
|---|---|
| (ca. 16 % Gesamt-Tensidgehalt) | |
| Natriumlaurylethersulfat 70%ig | 11,1 Gew.-% |
| Acylaminopolyglykolehtersulfat-Triethanolaminsalz 40%ig | 10,0 Gew.-% |
| Fettsäureamidoethyl-2-hydroxyethylcarboxymethylammoniumlaurylsulfat(amphoteres Glycin-Derivat) 38 %ig | 8,0 Gew.-% |
| Diglycerinmonolaurat | 2,0 Gew.-% |
| Natriumchlorid | 1,7 Gew.-% |
| Parfüm | 0,3 Gew.-% |
| Konservierungsmittel | 0,05 Gew.-% |
| Milchsäure | 0,1 Gew.-% |
| Wasser, vollentsalzt | 66,75 Gew.-% |

Viskosität: 3.000 mPas
Schaumzahlen nach Ross/Miles DIN 53902
1 g/l WAS, 40 °C, dest. $H_2O$ : 184/172

| 5. Körperreinigungs- und Körperwaschmittel Formulierung ohne Natriumlaurylethersulfat | |
|---|---|
| (mit ca. 20 % Gesamt-Tensidgehalt) | |
| Amphoteres Glycin-Derivat 38%ig (siehe Beispiel 4) | 20 Gew.-% |
| Sulfobernsteinsäureester 40%ig (siehe Beispiel 3) | 20 Gew.-% |
| Diglycerinmonolaureat | 5 Gew.-% |
| Ammoniumchlorid | 0,5 Gew.-% |
| Wasser voll entsalzt | 54,4 Gew.-% |

Viskosität: 2.450 mPas
Schaumzahlen nach Ross/Miles DIN 53902
1 g/l WAS, 40 °C, dest. $H_2O$ : 180/171

| 6. Mildes Haarshampoo | |
|---|---|
| (mit ca. 17 Gew.-% Gesamt-Tensidgehalt) | |
| Natriumlaurylethersulfat 70%ig | 14 Gew.-% |
| Acylaminopolyglykolethersulfat Triethanolaminsalz 40%ig | 10 Gew.-% |
| amphoteres Glycin-Derivat (s. Beispiel 4) 38 %ig | 6,3 Gew.-% |
| Tetraglycerindiisostearat | 1,5 Gew.-% |
| Natriumchlorid | 2,5 Gew.-% |
| Parfüm | 0,3 Gew.-% |
| Konservierungsmittel | 0,05 Gew.-% |
| Wasser vollentsalzt | 65,35 Gew.-% |

7

Viskosität: 1.800 mPas
Schaumzahlen nach Ross/Miles DIN 53902
1 g/l WAS, 40 °C, dest. $H_2O$ : 135/125

| 7. Geschirrspülmittel | |
| --- | --- |
| (mit ca. 20 % Gesamt-Tensidgehalt) | |
| Natriumalkylbenzosulfonat 50%ig | 23,8 Gew.-% |
| Natriumlaurylethersulfat 70%ig | 7,3 Gew.-% |
| Diglycerinmonolaurat | 3,0 Gew.-% |
| Parfüm | 0,15 Gew.-% |
| Konservierungsmittel | 0,05 Gew.-% |
| Wasser vollentsalzt | 65,7 Gew.-% |

Viskosität: 1.300 mPas
Schaumzahlen nach Ross/Miles DIN 53902
1 g/l WAS, 40 °C, dest. $H_2O$ : 201/189

| 8. Autoshampoo | |
| --- | --- |
| (mit ca. 20 % Gesamt-Tensidgehalt) | |
| Triethanolammonium-Alkylbenzolsulfonat 50 %ig | 25 Gew.-% |
| Natriumlaurylethersulfat 70%ig | 8 Gew.-% |
| Diglycerinmonolaurat | 2 Gew.-% |
| Natriumchlorid | 1 Gew.-% |
| Parfüm | 0,15 Gew.-% |
| Konservierungsmittel | 0,05 Gew.-% |
| Wasser vollentsalzt | 63,8 Gew.-% |

Viskosität: 2.400 mpas
Schaumzahlen nach Ross/Miles DIN 53902
1 g/l WAS, 40 °C, dest. $H_2O$ : 161/151

**Ansprüche**

1. Wasch-, Reinigungs- und/oder Körperreinigungsmittel, enthaltend mindestens ein nichtionogenes, aus einem Fettsäureester bestehendes Tensid und mindestens ein ionogenes und/oder amphoteres Tensid, dadurch gekennzeichnet, daß das Tensidgemisch mindestens einen Monofettsäureester mit
$C_8$ bis $C_{18}$
des Diglycerins und/oder einen Difettsäureester mit
$C_8$ bis $C_{18}$
des Tetraglycerins als Mischungsbestandteil enthält.

2. Wasch-, Reinigungs- und/oder Körperreinigungsmittel nach Anspruch 1, dadurch gekennzeichnet, daß das Tensidgemisch
2 bis 30 Gew.-%, vorzugsweise
10 bis 20 Gew.-%,
mindestens eines Monofettsäureesters des Diglycerins und/oder Difettsäureesters des Tetraglycerins, bezogen auf den Gesamttensidgehalt (100 Gew.-%) enthält.

3. Wasch-, Reinigungs- und/oder Körperreinigungsmittel nach Ansprüchen 1 und 2, dadurch gekennzeichnet, daß das Wasch-, Reinigungs- und/oder Körperreinigungsmittel Elektrolyte, vorzugsweise anorganische Chloride, in Gewichtsmengen von
0,1 bis 8 Gew.-%, vorzugsweise
0,7 bis 3 Gew.-%,

bezogen auf das Gesamtgewicht des Wasch-, Reinigungs- und/oder Körperreinigungsmittels enthält.

4. Wasch-, Reinigungs- und/oder Körperreinigungsmittel nach einem oder mehreren der Ansprüche 1 bis 3, dadurch gekennzeichnet, daß der Gesamttensidgehalt des Mittels

2,5 bis 60 Gew.-%, vorzugsweise

7 bis 35 Gew.-%

beträgt und der Restbestandteil aus Lösungs- und/oder Verdünnungsmitteln, vorzugsweise Wasser, niedrigen Alkoholen mit

$C_1$ bis $C_8$, vorzugsweise

$C_2$ bis $C_4$,

Diole mit

$C_2$ bis $C_8$, vorzugsweise

$C_3$ bis $C_6$,

und/oder Glycerin oder Glycerinderivaten und mindestens einem Elektrolyten sowie ggf. Zusatzmitteln, vorzugsweise Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln besteht.

5. Wasch-, Reinigungs- und/oder Körperreinigungsmittel nach einem oder mehreren der Ansprüche 1 bis 4, dadurch gekennzeichnet, daß der Gesamttensidgehalt des Mittels

2,5 bis 60 Gew.-%, vorzugsweise

7 bis 35 Gew.-%,

beträgt, wobei das Tensidgemisch aus

2 bis 30 Gew.-%, vorzugsweise

10 bis 20 Gew.-%,

mindestens eines Monofettsäureesters des Diglycerins und/oder Difettsäureesters des Tetraglycerins mit $C_{12}$ bis $C_{16}$ (bezogen auf den Gesamttensidgehalt 100 Gew.-%) besteht, und das Mittel zusätzlich

7 bis 0,1 Gew.-%, vorzugsweise

3 bis 0,3 Gew.-%,

mindestens eines Elektrolyten, vorzugsweise bestehend aus oder enthaltend Natriumchlorid,

90,5 bis 39,9 Gew.-%, vorzugsweise

90 bis 64,7 Gew.-%,

eines Lösungs- und/oder Verdünnungsmittels, vorzugsweise Wasser, niedrige Alkohole mit

$C_1$ bis $C_8$, vorzugsweise

$C_2$ bis $C_4$,

und/oder Diole mit

$C_3$ bis $C_8$, vorzugsweise

$C_3$ bis $C_6$,

und Zusatzmittel in Gewichtsmengen von

0,005 bis 12 Gew.-Teile,

(bezogen auf 100 Gew.-Teile des, aus dem Tensidgemisch, Elektrolyten und Lösungs- und/oder Verdünnungsmittel bestehenden Wasch-, Reinigungs- und/oder Körperreinigungsmittels), vorzugsweise

0,1 bis 5 Gew.-Teile,

vorzugsweise bestehend aus Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln, enthält.

6. Wasch-, Reinigungs- und/oder Körperreinigungsmittel nach einem oder mehreren der Ansprüche 1 bis 5, dadurch gekennzeichnet, daß das Tensidgemisch 2 bis 30 Gew.-% Diglycerin-Monolaurat (bezogen auf den Gesamttensidgehalt 100 Gew.-%) enthält.

7. Wasch-, Reinigungs- und/oder Körperreinigungsmittel nach einem oder mehreren der Ansprüche 1 bis 6, dadurch gekennzeichnet, daß das ionogene Tensid aus mindestens einem Alkylethersulfat, Alkylsulfat, Alkylarylsulfonat, Acylaminopolyglykolether-Sulfat, Olefinsulfonat, Paraffinsulfonat, Sulfo-Bernstein säureester und/oder Fettalkoholether-Carboxylat besteht oder als amphoteres Tensid Alkylamido-Betain und/oder ein amphoteres Glycerinderivat im Mittel enthalten ist.

8. Wasch-, Reinigungs- und/oder Körperreinigungsmittel nach einem oder mehreren der Ansprüche 1 bis 6, dadurch gekennzeichnet, daß das ionogene Tensid als Kation $Na^+$, $K^+$, $Mg^{++}$, $NH_4^+$, Alkylammonium, Alkanolammonium, vorzugsweise Monoethanolammonium, Triethanolammonium, $Na^+$ und/oder $NH_4^+$, enthält.

9. Wasch-, Reinigungs- und/oder Körperreinigungsmittel nach einem oder mehreren der Ansprüche 1 bis 8, dadurch gekennzeichnet, daß das Wasch-, Reinigungsund/oder Körperreinigungsmittel einen Gesamttensidgehalt von 2,5 bis 60 Gew.-%, vorzugsweise 7 bis 35 Gew.-%, (bezogen auf 100 Gew.-% des Mittels), aufweist, wobei das Tensidgemisch 2 bis 30 Gew.-%, vorzugsweise 10 bis 20 Gew.-%, mindestens eines Monofettsäureesters des Diglycerins und/oder Difettsäureesters des Tetraglycerins, bezogen auf den Gesamttensidgehalt (100 Gew.-%) und 98 bis bis 70 Gew.%, vorzugsweise 90 bis 80, mindestens eines ionogenen Tensides, bestehend aus mindestens einem Alkylaryl- oder Alkylethersulfat, Alkylsulfat, Alkylethersulfonat, Alkylhydroxyethersulfonat, Polyhydroxyalkylethersulfonat, Alkylarylsulfonat, vorzugsweise Alkylbenzolsulfonat, Acylaminopolyglykolether-Sulfat, Olefinsulfonat, Paraffinsulfonat, Sulfo-Bernsteinsäureester und/oder Fettalkoholether-Carboxylat und/oder als amphoteres Tensid Alkylamido-Betain und/oder amphoteres Glycerinderivat enthält, vorzugsweise jedoch ein ionogenes Tensid oder Tensidgemisch enthaltend oder bestehend aus Alkylethersulfat, Alkylhydroxyethersulfonat, Alkylsulfonat und/oder Alkylsulfat, das als Kation $Na^+$, $K^+$, $Mg^{++}$, $NH_4^+$, Monoethanolammonium und/oder Triethanolammonium besitzt, und der Restbestandteil aus Lösungs- und/oder Verdünnungsmitteln, vorzugsweise Wasser, niedrige Alkohole mit $C_1$ bis $C_8$, vorzugsweise $C_2$ bis $C_4$, und/oder Glycerin oder Glycerinderivate, Elektrolyte sowie ggf. Zusatzmittel, vorzugsweise Konservierungs-, Parfümierungs-, Färbemittel, pharmazeutische Wirkstoffe, Stellmittel zur pH-Regulierung, Komplexierungsmittel zur Maskierung von Metallionen, Hautpflegemittel und/oder Verdickungsmittel enthält.

10. Verwendung des Wasch-, Reinigungs- und/oder Körperreinigungsmittels nach einem oder mehreren der Ansprüche 1 bis 8, als Dusch-, Schaumbademittel, flüssiges Handreinigungsmittel oder Haarshampoo.

11. Verfahren zur Herstellung von Wasch-, Reinigungsund/oder Körperreinigungsmitteln enthaltend mindestens ein ionogenes und ein nichtionogenes Tensid, von denen das nichtionogene Tensid unter Verwendung von Fettsäurealkylestern, Mono- oder Polyhydroxyfettsäurealkylestern mit $C_8$ - $C_{18}$ in der Fettsäurekomponente und $C_1$ - $C_4$ in der Esterkomponente, die im alkalischen Medium mit einem oder mehreren Isopropylidenderivaten eines Polyglycerins umgesetzt werden, hergestellt wird, dadurch gekennzeichnet, daß diese Umsetzung mit einem Di- und/oder Tetraglycerin bei Temperaturen von

140 - 220 °C, vorzugsweise

170 - 200 °C,

und im Vakuum

bei 950 - 5 mbar, vorzugsweise

bei 500 - 10 mbar,

durchgeführt, der dabei entstehende $C_1$ - $C_4$ Alkohol durch Destillation entfernt, vorzugsweise kontinuierlich entfernt und das nachfolgend gereinigte, vorzugsweise durch Filtration, Zentrifugation, Destillation und/oder fraktionierte Destillation gereinigte Reaktionsprodukt hydrolisiert wird, indem mindestens eine Isopropylidengruppe des Reaktionsproduktes bei

20 bis 100 °C, vorzugsweise

50 bis 80 °C,

sowie bei Normal-, Unter- oder Überdruck durch saure Hydrolyse gespalten wird, die entstandenen Monofettsäureester des Diglycerins und/oder Tetraglycerins bei Temperaturen von

1 - 80 °C, vorzugsweise

10 - 40 °C,

und Druck oder Unterdruck von

0,1 bis 1,5 bar, vorzugsweise

0,3 bis 1,2 bar,

mit mindestens einem ionogenen Tensid, vorzugsweise mindestens einem Alkylethersulfat, Alkylsulfat, Alkylbenzolsulfonat, Acylaminopolyglykolether-Sulfat, Olefinsulfonat, Paraffinsulfonat, Alkylhydroxyethersulfonat, Polyhydroxyalkylethersulfonat, Sulfo-Bernsteinsäureester, Alkylamido-Betain, amphoteren Glycinderivat und/oder Fettalkoholether-Carboxylat, und/oder den anderen Bestandteilen oder einem Teil derselben vermischt und nachfolgend die restlichen Bestandteile hinzugefügt werden.

12. Verfahren nach Anspruch 11, dadurch gekennzeichnet, daß ein Überschuß von Diglycerin zur Umsetzung eingesetzt und/oder ein entstehendes Gemisch an Diund Monofettsäureestern des Diglycerins destillativ aufgetrennt wird.

13. Verfahren nach Ansprüchen 11 und 12, dadurch gekennzeichnet, daß das Wasch-, Reinigungsund/oder Körperreinigungsmittel auf einem pH-Wert von

2 bis 8, vorzugsweise

4 bis 7,

eingestellt wird.

10